(19) Europäisches Patentamt
European Patent Office
Office européen des brevets

(11) Publication number: **0 095 192 B1**

(12) **EUROPEAN PATENT SPECIFICATION**

(45) Date of publication of patent specification: **10.08.88**

(51) Int. Cl.$^4$: **C 07 D 209/48,** A 01 N 43/38

(21) Application number: **83105183.4**

(22) Date of filing: **25.05.83**

(54) **Tetrahydrophthalimides, and their production and use.**

(30) Priority: **26.05.82 JP 90387/82**
**21.09.82 JP 165445/82**

(43) Date of publication of application:
**30.11.83 Bulletin 83/48**

(45) Publication of the grant of the patent:
**10.08.88 Bulletin 88/32**

(84) Designated Contracting States:
**CH DE FR GB LI**

(56) References cited:
**EP-A-0 049 508**
**EP-A-0 049 511**
**EP-A-0 061 741**
**GB-A-2 046 754**
**CHEMICAL ABSTRACTS, vol. 94, no. 17, 27th April 1981, page 245, no. 133973g, Columbus, Ohio, US, H. OHTA et al.: "Quantitative structure-activity study of herbicidal N-aryl-3,4,5,6-tetrahydrophthalim ides and related cyclic imides"**

**The file contains technical information submitted after the application was filed and not included in this specification**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Nagano, Eiki**
**4-1-401, Ryodo-cho**
**Nishinomiya Hyogo (JP)**
Inventor: **Hashimoto, Shunichi**
**10-4-442, Sonehigashi-machi 2-chome**
**Toyonaka Osaka (JP)**
Inventor: **Yoshida, Ryo**
**5-19, Azajizoyama Higashiuneno**
**Kawanishi Hyogo (JP)**
Inventor: **Matsumoto, Hiroshi**
**10-2-211, Sonehigashi-machi 2-chome**
**Toyonaka Osaka (JP)**
Inventor: **Kamoshita, Katsuzo**
**3-11, Kofudai 2-chome Toyono-cho**
**Toyono-gun Osaka (JP)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 095 192 B1**

Courier Press, Leamington Spa, England.

## Description

The present invention relates to N-(2-fluoro-4-chloro-5-substituted phenyl)-3,4,5,6-tetrahydrophthalimide derivatives (hereinafter referred to as "tetrahydrophthalimide(s)"), and their production and use.

The said tetrahydrophthalimides are representable by the formula:

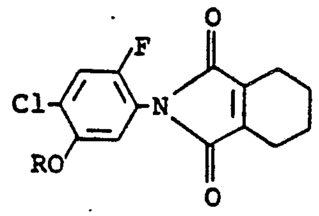

(I)

wherein R is a $C_1$—$C_8$ alkylaminocarbonyl($C_1$—$C_8$)alkyl group, a di($C_1$—$C_8$)alkylaminocarbonyl($C_1$—$C_8$)alkyl group, a $C_1$—$C_8$ alkyl($C_1$—$C_8$)alkoxyaminocarbonyl($C_1$—$C_8$)alkyl group, a hydroxyimino($C_1$—$C_8$)alkyl group, a $C_1$—$C_8$ alkoxyimino($C_1$—$C_8$)alkyl group, a $C_1$—$C_8$ alkoxycarbonyl($C_1$—$C_8$)alkoxyimino($C_1$—$C_8$)alkyl group or a $C_1$—$C_8$ alkenoxyimino($C_1$—$C_8$)alkyl group.

It is known that certain kinds of N-phenyltetrahydrophthalimides are effective as herbicides. For instance, the herbicidal use of 2-fluoro-4-chlorophenyl-3,4,5,6-tetrahydrophthalimide, 4-chloro-3-(2-hydroxyiminopropoxy)phenyl-3,4,5,6-tetrahydrophthalimide, etc. is disclosed in U.S. patent 4,032,326, EP 0049511A, EP—A—49508, etc. However, their herbicidal effect is still not always satisfactory.

It has now been found that the tetrahydrophthalimides (I) show a strong herbicidal activity against a wide variety of weeds including Gramineae weeds, Cyperaceae weeds and broad-leaved weeds at small doses and do not produce any material phytotoxicity on various agricultural crops. Examples of Gramineae weeds against which the tetrahydrophthalimides (I) show a herbicidal activity are barnyardgrass (*Echinochloa crus-galli*), green foxtail (*Setaria viridis*), large crabgrass (*Digitaria sanguinalis*), Johnsongrass (*Sorghum halepense*), wild oat (*Avena fatua*), water foxtail (*Alopecurus geniculatus*), goosegrass (*Eleusine indica*), annual bluegrass (*Poa annua*), etc. Examples of Cyperaceae weeds are purple nutsedge (*Cyperus rotundus*, hardstem bulrush (*Scirpus juncoides*), nutsedge (*Cyperus serotinus*), slender spikerush (*Eleocharis acicularis*), etc. Examples of broad-leaved weeds are tall morningglory (*Ipomoea purpurea*), velvetleaf (*Abutilon theophrasti*), sicklepod (*Cassia obtusifolia*), wild sunflower (*Helianthus annus*), cocklebur (*Xanthium pennsylvanicum*), wild mustard (*Brassica kaber*), common purslane (*Portulaca oleracea*), jimsonweed (*Datura stramonium*), hemp sesbania (*Sesbania exaltata*), sun spurge (*Euphorbia helioscopia*), prickly sida (*Sida spinosa*), common ragweed (*Ambrosia artemisifolia*), smartweed sp. (*Polygonum* sp.), redroot pigweed (*Amaranthus retroflexus*), bedstraw (*Galium aparine*), scentless chamomile (*Matricaria inodora*), birdseye speedwell (*Veronica persica*), wild buckwheat (*Polygonum convolvulus*), ladysthumb (*Polygonum pensylvanicum*), common lambsquarters (*Chenopodium album*), black nightshade (*Solanum nigrum*), monochoria (*Monochoria vaginalis*), American waterwort (*Elatine americana*), false pimpernel (*Lindernia procumbens*), toothcup (*Rotala indica*), arrowhead (*Sagittaria pygmaea*), etc.

Accordingly, the tetrahydrophthalimides (I) can be used as herbicides applicable for field crops and vegetables as well as paddy rice. They are also useful as herbicides to be employed for orchard, lawn, pasture, tea garden, mulberry field, rubber plantation, forest, etc.

The tetrahydrophthalimides (I) can be produced by reacting a hydroxyphenyltetrahydrophthalimide of the formula:

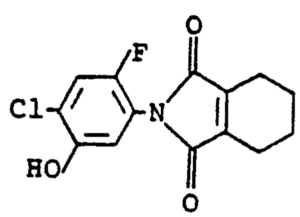

(II)

with a halide of the formula:

RY

wherein Y is a chlorine atom or a bromine atom and R is as defined above, usually in an inert solvent (e.g. dimethylformamide, dimethylsulfoxide) in the presence of a base such as an alkali metal carbonate (e.g. potassium carbonate), an alkali metal hydroxide (e.g. potassium hydroxide), an alkali metal hydride (e.g. sodium hydride), an alkali metal alkoxide (e.g. sodium methoxide, sodium ethoxide) or a tertiary amine (e.g. pyridine, triethylamine). The reaction temperature is normally from 0 to 100°C. The molar ratio of the hydroxyphenyltetrahydrophthalimide (II) and the halide is preferred to be from 1:1.0 to 1:10.

The thus produced tetrahydrophthalimide (I) may be, when desired, purified by a per se conventional procedure such as recrystallization or column chromatography.

The starting hydroxyphenyltetrahydrophthalimide (II) can be produced from a 2-chloro-4-fluorophenol according to the method as disclosed in EP 0061741A.

A practical and presently preferred embodiment for production of the tetrahydrophthalimides (I) is illustratively shown in the following Example.

## Example 1

To a solution of N-(4-chloro-2-fluoro-5-hydroxyphenyl)-3,4,5,6-tetrahydrophthalimide (5.9 g) in dimethylformamide (20 ml) was added potassium carbonate (1.5 g), and the resultant mixture was heated to 30—40°C, followed by addition of α-bromo-N,N-dimethylacetamide (3.4 g). The reaction mixture was stirred at 60—70°C for 4 hours and extracted with toluene. The extract was washed with water, dried over anhydrous magnesium sulfate. Toluene was removed in vacuo, and the residue was crystallized from methanol to obtain 5 g of N-(4-chloro-2-fluoro-5-dimethylaminocarbonylmethoxyphenyl)-3,4,5,6-tetrahydrophthalimide. M.P., 160—161°C.

Examples of the tetrahydrophthalimides (I) produced by the same procedure as above are shown in Table 1.

Table 1

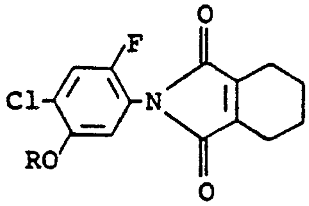

(I)

| Compound No. | R | Physical constant |
|---|---|---|
| 1 | $-CH_2CONHCH_3$ | M.P. 190-191°C |
| 2 | $-CH_2CON(CH_3)_2$ | M.P. 160-161°C |
| 3 | 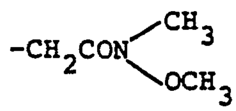 | $n_D^{22.5}$ 1.5176 |
| 4 | 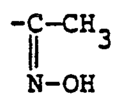 | M.P. 131-131.5°C |
| 5 | 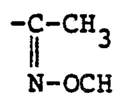 | M.P. 137.5-138°C |
| 6 | 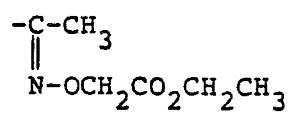 | Glassy |
| 7 | 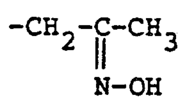 | Glassy |
| 8 | 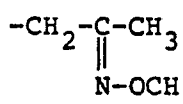 | $n_D^{18.5}$ 1.5575 |
| 9 | 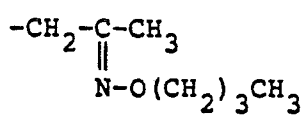 | $n_D^{23}$ 1.5393 |
| 10 | 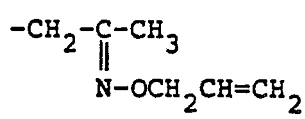 | $n_D^{25.5}$ 1.5501 |
| 11 | 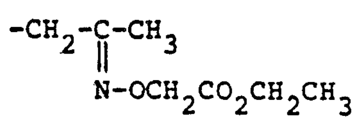 | $n_D^{25.5}$ 1.5355 |

In the practical usage of the tetrahydrophthalimides (I), they may be applied as such or in any composition such as emulsifiable concentrates, wettable powders, suspensions, granules or dusts.

The content of the tetrahydrophthalimides (I) as the active ingredient in such composition is usually within a range of 0.1 to 95% by weight, preferably of 1 to 80% by weight.

In formulation of those compositions, a solid or liquid carrier or diluent may be used. As the solid carrier or diluent, there may be employed fine dust or granules of kaolin clay, attapulgite clay, bentonite, terra abla, pyrophyllite, talc, diatomaceous earth, calcite, walnut powder, urea, ammonium sulfate, synthetic hydrated silicon dioxide, etc. As the liquid carrier or diluent, there may be employed aromatic hydrocarbons (e.g. xylene, methylnaphthalene), alcohols (e.g. isopropanol, ethylene glycol, cellosolve), ketones (e.g. acetone, cyclohexanone, isophorone), plant oils (e.g. soybean oil, cotton seed oil), dimethylsulfoxide, acetonitrile, water, etc.

A surface active agent used for emulsification, dispersion or spreading may be any of the non-ionic, anionic, cationic and amphoteric type of agents. Examples of the surface active agent include alkylsulfates, alkylaryl sulfonates, dialkylsuccinates, polyoxyethylene alkylaryl phosphates, polyoxyethylene alkyl ethers, polyoxyethylene alkylaryl ethers, polyoxyethylene-polyoxypropylene blocked polymers, sorbitan fatty acid esters, polyoxyethylene sorbitan fatty acid esters, polyoxyethylene resin acid esters, abietic acid, dinaphthylmethanedisulfonates, paraffin and the like. If necessary, ligninsulfonates, alginates, polyvinyl alcohol, gum arabic, CMC (carboxymethylcellulose), PAP (isopropyl acid phosphate) or the like may be used as an auxiliary agent.

Practical embodiments of the herbicidal composition according to the invention are illustratively shown in the following examples wherein part(s) and % are by weight. The compound number of the active ingredient corresponds to the one in Table 1.

Formulation Example 1

Fifty parts of Compound No. 4, 2 parts of alkylsulfate, 3 parts of ligninsulfonate and 45 parts of synthetic hydrated silicon dioxide are well mixed while being powdered to obtain a wettable powder.

Formulation Example 2

Ten parts of Compound No. 5, 14 parts of polyoxyethylene alkylaryl ether, 6 parts of alkylarylsulfonate and 40 parts of isophorone and 30 parts of xylene are well mixed to obtain an emulsifiable concentrate.

Formulation Example 3

Two parts of Compound No. 10, 1 part of synthetic hydrated silicon dioxide, 30 parts of bentonite and 67 parts of kaolin clay are well mixed while being powdered. The mixture is then kneaded with water, granulated and dried to obtain a granule.

Formulation Example 4

Three parts of Compound No. 1, 0.5 part of isopropyl acid phosphate, 66.5 parts of kaolin clay and 30 parts of talc are well mixed while being powdered to obtain a dust.

Formulation Example 5

Twenty parts of Compound No. 2 is mixed with 60 parts of an aqueous solution containing 3% of polyoxyethylene sorbitan monooleate and pulverized until the particle size of the solid component in the mixture becomes less than 3 microns. Twenty parts of an aqueous solution containing 3% of sodium alginate as a dispersing agent are incorporated therein to obtain a suspension.

These compositions comprising the tetrahydrophthalimides (I) may be applied as such, or after diluted with water, to weeds in suitable application modes such as spraying, perfusion, etc. For instance, they may be spread over, perfused into or admixed with soil. Further, for instance, they may be applied for foliar treatment. If necessary, they may be used together with other herbicides to improve their activity as herbicides, and in some cases, a synergistic effect can be expected. They may be also applied in combination with insecticides, acaricides, nematocides, fungicides, plant growth regulators, fertilizers, soil controlling agents, etc.

A dosage rate of the tetrahydrophthalimide (I) as the active ingredient may be generally from 0.01 to 100 grams, preferably from 0.1 to 50 grams, per are. In the practical usage of the tetrahydrophthalimide (I) as emulsifiable concentrates, wettable powders or suspensions, it may be diluted with 1 to 10 liters of water (optionally including an auxiliary agent such as a spreading agent) per are. When formulated into granules or dust, it may be used as such without dilution.

The application of the tetrahydrophthalimides (I) as herbicides will be illustratively shown in the following Examples wherein the phytotoxicity to cultivated plants and the herbicidal activity on weeds were evaluated as follows: the aerial parts of the test plants were cut off and weighed (fresh weight); the percentage of the fresh weight of the treated plant to that of the untreated plant was calculated with the latter fresh weight taken as 100; and the phytotoxicity and the herbicidal activity were evaluated by the standard given in the table below.

| Rating value | Fresh weight (percentage to untreated plant) (%) | |
|---|---|---|
| | Herbicidal activity | Phytotoxicity |
| 0 | 91 - | 91 - |
| 1 | 71 - 90 | 71 - 90 |
| 2 | 41 - 70 | 51 - 70 |
| 3 | 11 - 40 | 31 - 50 |
| 4 | 1 - 10 | 11 - 30 |
| 5 | 0 | 0 - 10 |

The following compounds were used in the Examples for comparison:

| Compound No. | Structure | Remarks |
|---|---|---|
| (a) | 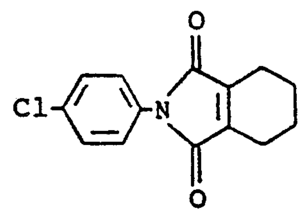 | U.S. patent 3,984,435 |
| (b) | 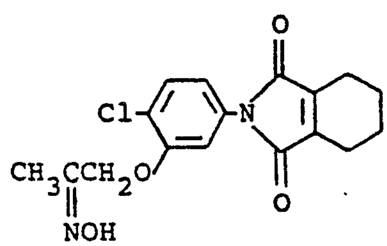 | EP 0049511A |
| (c) | 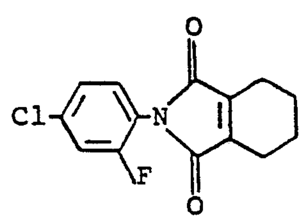 | U.S. patent 4,032,326 |

Test Example 1

Trays (33 × 23 $cm^2$) were filled with upland field soil, and the seeds of corn, wheat, soybean, cocklebur, tall morningglory, velvetleaf, black nightshade and hemp sesbania were sowed therein and grown for 18 days in a greenhouse. A designed amount of the test compound formulated into an emulsifiable concentrate and diluted with water was sprayed to the foliage of the test plants over the top by means of a small hand sprayer at a spray volume of 5 liters per are. At the time of application, the test plants were generally at the 1 to 4 leaf stage and had a height of 1.5 to 12 cm. Twenty days thereafter, phytotoxicity and herbicidal activity were examined. The results are shown in Table 2.

Table 2

| Compound No. | Dosage (weight of active ingredient, g/are) | Phytotoxicity | | | Herbicidal activity | | | | |
|---|---|---|---|---|---|---|---|---|---|
| | | Corn | Wheat | Soybean | Cocklebur | Tall morning-glory | Velvet-leaf | Black night-shade | Hemp ses-bania |
| 1 | 0.63 | 1 | 1 | - | - | 5 | 5 | 5 | 5 |
| 2 | 0.63 | 1 | 0 | - | - | 5 | 5 | 5 | 5 |
| 3 | 0.63 | 1 | 1 | - | - | 5 | 5 | 5 | 5 |
| 4 | 1.25 | 1 | 1 | 3 | 5 | 5 | 5 | 5 | 5 |
| | 0.32 | 0 | 0 | 2 | 4 | 3 | 5 | 4 | 4 |
| 6 | 1.25 | 1 | 0 | 4 | 5 | 5 | 5 | 5 | 5 |
| | 0.32 | 0 | 0 | 2 | 5 | 5 | 5 | 5 | 5 |
| 7 | 0.63 | 2 | 1 | 4 | 5 | 5 | 5 | 5 | 5 |
| | 0.16 | 0 | 0 | 2 | 5 | 5 | 5 | 5 | 5 |
| 9 | 0.63 | 1 | 0 | 2 | 5 | 5 | 5 | 5 | 5 |
| | 0.16 | 0 | 0 | 1 | 4 | 5 | 5 | 5 | 5 |
| 10 | 0.63 | 2 | 1 | 4 | 5 | 5 | 5 | 5 | 5 |
| | 0.16 | 0 | 0 | 2 | 4 | 5 | 5 | 5 | 5 |
| 11 | 0.63 | 1 | 2 | 5 | 5 | 5 | 5 | 5 | 5 |
| | 0.16 | 0 | 0 | 3 | 4 | 5 | 5 | 5 | 5 |
| (a) | 1.25 | 1 | 1 | 1 | 3 | 2 | 5 | 3 | 0 |
| | 0.63 | 0 | 0 | 0 | - | 1 | 5 | 1 | 0 |
| | 0.32 | 0 | 0 | 0 | 0 | 1 | 4 | 1 | 0 |
| (b) | 1.25 | 0 | 1 | 1 | 2 | 1 | 4 | 3 | 0 |
| | 0.63 | 0 | 0 | 0 | 0 | 0 | 2 | 1 | 0 |
| (c) | 0.32 | - | - | 3 | 3 | 3 | 4 | 3 | 1 |

Test Example 2

Trays (33 × 23 $cm^2$) were filled with field soil, and the seeds of soybean, cotton, corn, wheat, tall morningglory, velvetleaf, jimsonweed, prickly sida, johnsongrass and scentless chamomile were sowed therein and covered with soil in 1 cm depth. A designed amount of the test compound formulated into an emulsifiable concentrate and diluted with water was sprayed to the soil surface at a spray volume of 10 liters per are. The test plants were grown outdoors for 3 weeks, and herbicidal activity and phytotoxicity were examined. The results are shown in Table 3.

Table 3

| Compound No. | Dosage (weight of active ingredient, g/are) | Phytotoxicity | | | | Herbicidal activity | | | | | |
|---|---|---|---|---|---|---|---|---|---|---|---|
| | | Soybean | Cotton | Corn | Wheat | Tall morning-glory | Velvetleaf | Jimsonweed | Prickly sida | Johnson grass | Scentless chamomile |
| 1 | 10 | 0 | - | 1 | 0 | 3 | 5 | 4 | 5 | 4 | 5 |
| 2 | 10 | 1 | - | 1 | - | 5 | 5 | 5 | 5 | 5 | 5 |
| | 2.5 | 0 | 0 | 0 | - | 3 | 5 | 5 | 5 | 5 | 5 |
| 3 | 10 | 0 | 1 | 0 | 1 | 4 | 5 | 5 | 5 | 2 | 5 |
| 4 | 20 | 1 | 2 | 3 | 5 | 4 | 5 | - | 5 | 5 | 5 |
| | 5 | 0 | 1 | 2 | 3 | 3 | 5 | - | 3 | 4 | 5 |
| 5 | 20 | 1 | 2 | 2 | 5 | 5 | 5 | - | 5 | 5 | 5 |
| | 5 | 0 | 1 | 1 | 3 | 3 | 5 | - | 5 | 5 | 5 |
| 6 | 20 | 2 | 4 | 2 | 3 | 5 | 5 | - | 5 | 5 | 5 |
| | 5 | 0 | 1 | 1 | 2 | 3 | 5 | - | 5 | 4 | 5 |
| 7 | 20 | 0 | 2 | 3 | 4 | 5 | 5 | - | 5 | 5 | 5 |
| | 5 | 0 | 1 | 0 | 2 | 3 | 4 | - | 4 | 5 | 5 |
| 8 | 20 | 1 | 2 | 2 | 3 | 5 | 5 | - | 5 | 5 | 5 |
| | 5 | 0 | 1 | 0 | 2 | 3 | 4 | - | 4 | 4 | 5 |
| 9 | 20 | 2 | 3 | 2 | 2 | 5 | 5 | - | 5 | 5 | 5 |
| | 5 | 1 | 1 | 1 | 1 | 3 | 3 | - | 4 | 3 | 3 |
| 10 | 20 | 0 | 3 | 2 | 3 | 4 | 5 | - | 5 | 5 | 5 |
| | 5 | 0 | 1 | 1 | 1 | 4 | 4 | - | 4 | 4 | 3 |
| 11 | 20 | 1 | 1 | 0 | 3 | 4 | 5 | - | 5 | 4 | 5 |
| | 5 | 0 | 0 | 0 | 2 | 3 | 4 | - | 3 | 3 | 5 |
| (a) | 20 | 1 | 1 | 0 | 2 | 1 | 4 | 1 | 1 | 1 | 3 |
| | 5 | 0 | 0 | 0 | 0 | 0 | 2 | 0 | 0 | 0 | 2 |
| (b) | 20 | 0 | 1 | 0 | 2 | 3 | 5 | - | 4 | 4 | 5 |
| | 5 | 0 | 0 | 0 | 0 | 1 | 2 | - | 2 | 1 | 3 |

Test Example 3

Wagner's pots (1/500 are) were filled with paddy field soil, and the seeds of barnyardgrass, broad-leaved weeds (e.g. false pimpernel, toothcup, waterwort), arrowhead and hardstem bulrush were sowed therein to a depth of 1 to 2 cm. The pots were placed under a flooded condition, and the rice seedlings of the 3-leaf stage were transplated therein at a depth of 1 to 2 cm and grown for 4 days in a greenhouse. A designed amount of the test compound formulated into an emulsifiable concentrate and diluted with water was perfused into the pots at a perfusion volume of 10 ml per pot. The test plants were further grown for 20 days in the greenhouse, and phytotoxicity and herbicidal activity were examined. The results are shown in Table 4.

Table 4

| Compound No. | Dosage (weight of active ingredient, g/are) | Phytotoxicity | Herbicidal activity | | | |
|---|---|---|---|---|---|---|
| | | Rice plant | Barnyardgrass | Broad-leaved weeds | Arrowhead | Hardstem bulrush |
| 10 | 5 | 1 | 5 | 5 | 5 | 5 |
| | 2.5 | 1 | 5 | 5 | 5 | 4 |
| | 1.25 | 0 | 5 | 5 | 5 | 3 |

**Claims**

1. A compound of the formula:

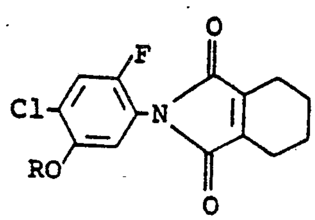

(I)

wherein R is a $C_1$—$C_8$ alkylaminocarbonyl($C_1$—$C_8$)alkyl group, a di($C_1$—$C_8$)alkylaminocarbonyl($C_1$—$C_8$)alkyl group, a $C_1$—$C_8$ alkyl($C_1$—$C_8$)alkoxyaminocarbonyl($C_1$—$C_8$)alkyl group, a hydroxyimino($C_1$—$C_8$)alkyl group, a $C_1$—$C_8$ alkoxyimino($C_1$—$C_8$)alkyl group, a $C_1$—$C_8$ alkoxycarbonyl($C_1$—$C_8$)alkoxyimino($C_1$—$C_8$)alkyl group or a $C_1$—$C_8$ alkenoxyimino($C_1$—$C_8$)alkyl group.

2. A herbicidal composition which comprises as an active ingredient a herbicidally effective amount of the compound according to claim 1 and an inert carrier.

3. A method for controlling or exterminating weeds which comprises applying a herbicidally effective amount of the compound according to claim 1 to the area where the weeds grow or will grow.

4. The method according to claim 3, wherein the area is a plowed field.

5. Use of the compound according to claim 1 as a herbicide.

**Patentansprüche**

1. Eine Verbindung der Formel

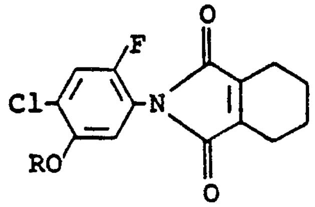

(I)

in der R einen $C_1$—$C_8$-Alkylaminocarbonyl-$C_1$—$C_8$-alkylrest, einen Di-$C_1$—$C_8$-alkylaminocarbonyl-$C_1$—$C_8$-alkylrest, einen $C_1$—$C_8$-Alkyl-$C_1$—$C_8$-alkoxyaminocarbonyl-$C_1$—$C_8$-alkylrest, einen Hydroxyimino-$C_1$—$C_8$-alkylrest, einen $C_1$—$C_8$-Alkoxyimino-$C_1$—$C_8$-alkylrest, einen $C_1$—$C_8$-Alkoxycarbonyl-$C_1$—$C_8$-alkoxyimino-$C_1$—$C_8$-alkylrest oder einen $C_1$—$C_8$-Alkenoxyimino-$C_1$—$C_8$-alkylrest bedeutet.

2. Herbizide Zusammensetzung, umfassend als Wirkstoff eine herbizid wirksame Menge einer Verbindung nach Anspruch 1 und einen inerten Träger.

3. Verfahren zur Bekämpfung oder Vertilgung von Unkräutern, das Anwendung einer herbizid wirksamen Menge einer Verbindung nach Anspruch 1 auf die Fläche, auf der die Unkräuter wachsen oder wachsen werden, umfaßt.

4. Verfahren nach Anspruch 3, wobei die Fläche ein gepflügtes Feld ist.

5. Verwendung einer Verbindung nach Anspruch 1 als Herbizid.

**Revendications**

1. Un composé de formule:

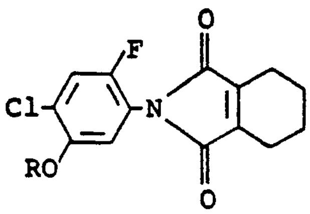

(I)

dans laquelle R est un groupe ($C_1$—$C_8$)alkylaminocarbonyl($C_1$—$C_8$)alkyle, un groupe di($C_1$—$C_8$)alkylaminocarbonyl($C_1$—$C_8$)alkyle, un groupe ($C_1$—$C_8$)alkyl($C_1$—$C_8$)alcoxyaminocarbonyl($C_1$—$C_8$)alkyle, un groupe hydroxyimino($C_1$—$C_8$)alkyle, un groupe($C_1$—$C_8$)alcoxyimino($C_1$—$C_8$)alkyle, un groupe($C_1$—$C_8$)alcoxycarbonyl($C_1$—$C_8$)alcoxyimino($C_1$—$C_8$)alkyle ou un groupe($C_1$—$C_8$)alcèneoxyimino($C_1$—$C_8$)alkyle.

2. Une composition herbicide qui comprend comme ingrédient actif une quantité herbicide d'un composé selon la revendication 1 et un support inerte.

3. Une méthode pour contrôler ou pour exterminer les mauvaises herbes qui consiste à appliquer une quantité herbicide du composé selon la revendication 1 à la surface où poussent ou pousseront les mauvaises herbes.

4. La méthode selon la revendication 3, selon laquelle la surface est un champ de labour.

5. Utilisation du composé selon la revendication 1 comme herbicide.